# FASCICULE DE BREVET EUROPEEN

(11) **EP 1 174 450 B1**
(45) Date de publication et mention de la délivrance du brevet: **16.03.2011**
(21) Numéro de dépôt: 01401818.8
(22) Date de dépôt: 06.07.2001
(51) Int. Cl.: A61K 8/00, C08G 18/08, C08G 18/28, C08G 18/32, C08G 18/48, C09D 7/00

(54) **Polyuréthanes associatifs cationiques et leur utilisation comme épaississants**
Assoziative kationische Polyurethane und ihre Verwendung als Verdickungsmittel
Associative cationic polyurethanes and their use as thickeners

(30) Priorité: 21.07.2000 FR 0009609
(43) Date de publication de la demande: 23.01.2002
(73) Titulaire: L'Oréal, 75008 Paris (FR)
(72) Inventeur: Mougin, Nathalie, 75011 Paris (FR); Cottard, François, 92300 Levallois-Perret (FR); De la Mettrie, Roland, 78110 Le Vesinet (FR); Lion, Bertrand, 95270 Luzarches (FR); Maury, Elise, 75003 Paris (FR)
(74) Mandataire: Dossmann, Gérard

(56) Documents cités:
- EP-A- 0 978 522
- US-A- 4 068 035
- US-A- 4 617 341

## Description

La présente invention concerne de nouveaux polyuréthannes associatifs cationiques amphiphiles, hydrosolubles ou hydrodispersibles, ainsi que leur utilisation dans des compositions pour application topique, notamment à usage cosmétique ou thérapeutique.

Le terme "hydrosoluble" ou "soluble dans l'eau" concernant les polyuréthannes associatifs de la présente invention signifie que ces polymères ont une solubilité dans l'eau à température ambiante au moins égale à 1 % en poids, c'est-à-dire que jusqu'à cette concentration, aucun précipité ne peut être détecté à l'oeil nu et la solution est parfaitement limpide et homogène.

On entend par polyuréthannes "hydrodispersibles" ou "dispersibles dans l'eau" des polymères qui, lorsqu'on les met en suspension dans l'eau, forment spontanément des globules ayant une taille moyenne, mesurée par diffusion de la lumière sur un appareil de type Coulter, comprise entre 5 nm et 600 nm, et en particulier entre 5 nm et 500 nm.

L'épaississement et/ou la gélification des milieux aqueux par des polymères est depuis longtemps un important sujet de recherche, notamment dans le domaine de la cosmétique et de la pharmacie. L'obtention d'un effet d'épaississement intéressant par un polymère hydrosoluble suppose généralement une masse molaire élevée et un volume hydrodynamique important. La gélification d'un milieu aqueux est alors considérée comme le résultat d'un réseau polymère tridimensionnel obtenu par une réticulation de polymères linéaires ou par copolymérisation de monomères bifonctionnels et polyfonctionnels. L'utilisation de tels polymères de masse molaire très élevée pose cependant un certain nombre de problèmes tels que la texture peu agréable et la difficulté d'étalement des gels obtenus.

Le document US 4,617,341 décrit un oligouréthanne cationique sous la forme d'un latex pour l'industrie du papier et du carton. Cet oligouréthanne possède une structure différente du polyuréthanne selon l'invention.

Le document US 4,068,035 décrit une composition de polyuréthanne hydrophile pour traiter des matériaux textiles.

Le document EP 0 978 522 décrit des épaississants non-ioniques polyuréthanne/urée associatif pour épaissir et modifier la rhéologie liquide de systèmes aqueux.

Une approche intéressante a consisté à utiliser comme épaississant des polymères capables de s'associer réversiblement entre eux ou avec d'autres molécules ou particules. Cette association physique donne lieu à des systèmes macromoléculaires thixotropes ou rhéofluidifiables, c'est-à-dire des systèmes dont la viscosité dépend des forces de cisaillement auxquelles ils sont soumis.

De tels polymères capables de s'associer réversiblement entre eux ou avec d'autres molécules sont appelés « polymères associatifs ». Les forces d'interactions mises en jeu peuvent être de nature très différente, par exemple de nature électrostatique, de type liaison hydrogène ou des interactions hydrophobes.

Un cas particulier de polymères associatifs sont des polymères amphiphiles, c'est-à-dire des polymères comportant une ou plusieurs parties hydrophiles qui les rendent solubles dans l'eau et une ou plusieurs zones hydrophobes par lesquelles les polymères interagissent et se rassemblent entre eux ou avec d'autres molécules.

On a déjà préconisé en cosmétique l'utilisation de polymères associatifs et en particulier de polyuréthannes associatifs. Cependant, les propriétés rhéologiques et cosmétiques de ces polymères ne sont pas optimales.

On a découvert une nouvelle famille de polyuréthannes amphiphiles associatifs cationiques, hydrosolubles ou hydrodispersibles, dont les qualités épaississantes sont excellentes et qui possèdent de bonnes propriétés cosmétiques.

Ses excellentes propriétés épaississantes permettent une utilisation du polymère en quantité réduite. Cet avantage permet une amélioration de la texture de la composition le contenant.

Le gel obtenu en utilisant les polyuréthannes associatifs de l'invention est agréable au toucher et s'étale facilement.

Un objet de la présente invention est donc une nouvelle famille de polyuréthannes associatifs amphiphiles cationiques, hydrosolubles ou hydrodispersibles.

Un autre objet de la présente invention est une composition cosmétique contenant au moins un polyuréthanne associatif amphiphile cationique, hydrosoluble ou hydrodispersible.

Un troisième objet de l'invention est l'utilisation de ces polyuréthannes à titre d'épaississants ou de gélifiants dans les compositions pour application topique à usage cosmétique ou thérapeutique.

D'autres objets apparaîtront à la lecture de la description et des exemples qui suivent.

La famille de polyuréthannes associatifs amphiphiles cationiques, hydrosolubles ou hydrodispersibles, conforme à l'invention peut être représentée par la formule générale (I) suivante :

R-X-(P)ₙ-[L-(Y)ₘ]-L'-(P')ₚ-X'-R' (I)

dans laquelle :
R et R', identiques ou différents, représentent un groupement hydrophobe ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000 ;
la molécule contenant au moins une fonction amine protonée ou quaternisée et au moins deux groupements hydrophobes

Dans un mode de réalisation préféré des polyuréthannes de la présente invention, les seuls groupements hydrophobes sont les groupes R et R' aux extrémités de chaîne.

Une famille préférée de polyuréthannes associatifs cationiques selon la présente invention est celle correspondant à la formule I ci-dessus dans laquelle
R et R' représentent tous les deux indépendamment un groupement hydrophobe,
X, X' représentent chacun un groupe L",
n et p valent entre 1 et 1000 et
L, L', L", P, P', Y et m ont la signification indiquée ci-dessus.

Une autre famille de polyuréthannes associatifs cationiques selon la présente invention est celle correspondant à la formule I ci-dessus dans laquelle R et R' représentent tous les deux indépendamment un groupement hydrophobe, X, X' représentent chacun un groupe L", n et p valent 0, et L, L', L", Y et m ont la signification indiquée ci-dessus.

Le fait que n et p valent 0 signifie que ces polymères ne comportent pas de motifs dérivés d'un monomère à fonction amine, incorporé dans le polymère lors de la polycondensation. Les fonctions amine protonées de ces polyuréthannes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Encore une autre famille préférée de polyuréthannes associatifs cationiques selon la présente invention est celle correspondant à la formule I ci-dessus dans laquelle
R et R' représentent tous les deux indépendamment un groupement hydrophobe,
X et X' représentent tous les deux indépendamment un groupement comportant une amine quaternaire,
n et p valent zéro, et
L, L', Y et m ont la signification indiquée ci-dessus.

La masse moléculaire moyenne en nombre des polyuréthannes associatifs amphiphiles cationiques de l'invention est comprise de préférence entre 400 et 500 000, en particulier entre 1000 et 400 000 et idéalement entre 1000 et 300 000.

Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.

Préférentiellement, le groupement hydrophobe hydrocarboné provient d'un composé monofonctionnel.

A titre d'exemple, le groupement hydrophobe peut être issu d'un alcool gras tel que l'alcool stéarylique, l'alcool dodécylique, l'alcool décylique. Le groupement hydrophobe peut également être un polymère hydrocarboné tel que par exemple le polybutadiène.

Lorsque X et/ou X' désignent un groupement comportant une amine tertiaire ou quaternaire, X et/ou X' peuvent représenter l'une des formules suivantes : 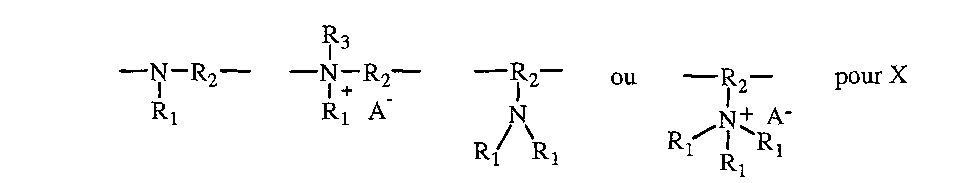 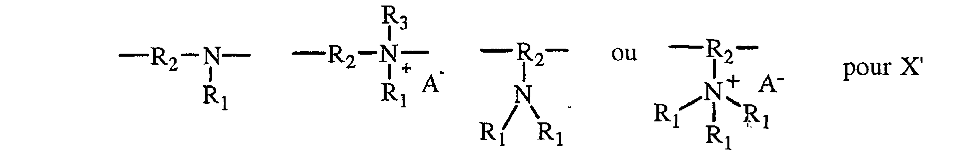 dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
A⁻ est un contre-ion physiologiquement acceptable.

Les groupements L, L' et L" représentent un groupe de formule : 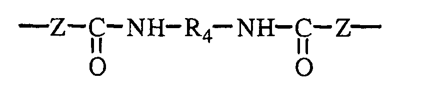 dans laquelle :
Z représente -O-, -S- ou -NH- ; et
R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

Les groupements P et P', comprenant une fonction amine peuvent représenter au moins l'une des formules suivantes : 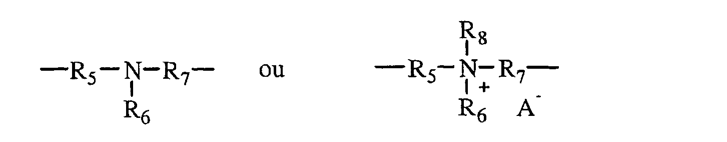 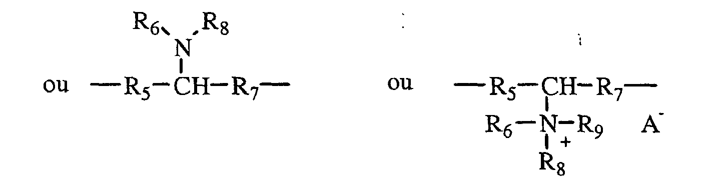 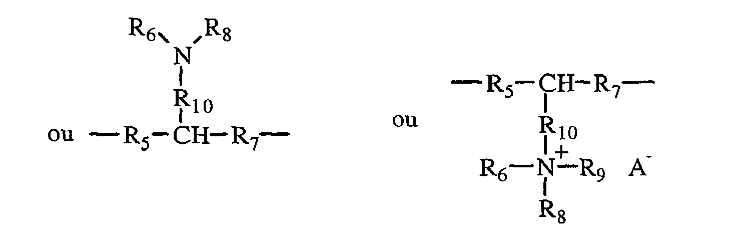 dans lesquelles :
R₅ et R₇ ont les mêmes significations que R₂ défini précédemment;
R₆, R₈ et R₉ ont les mêmes significations que R₁ et R₃ définis précédemment ;
R₁₀ représente un groupe alkylène, linéaire ou ramifié, éventuellement insaturé et pouvant contenir un ou plusieurs hétéroatomes choisis parmi N, O, S et P,
et A- est un contre-ion physiologiquement acceptable.

En ce qui concerne la signification de Y, on entend par groupement hydrophile, un groupement hydrosoluble polymérique ou non.

A titre d'exemple, on peut citer, lorsqu'il ne s'agit pas de polymères, l'éthylèneglycol, le diéthylèneglycol et le propylèneglycol.

Lorsqu'il s'agit, conformément à un mode de réalisation préféré de l'invention, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthannes amphiphiles associatifs cationiques de formule I selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthannes, des polyurées et des polythiourées. Le terme "polyuréthannes" choisi pour désigner les nouveaux polymères associatifs de la présente invention englobe ces trois types de polymères à savoir les polyuréthannes proprement dits, les polyurées et les polythiourées ainsi que des copolymères de ceux-ci.

Un premier type de composés entrant dans la préparation du polymère de formule I de l'invention est un composé comportant au moins un motif à fonction amine. Ce composé peut être multifonctionnel, mais préférentiellement le composé est difonctionnel, c'est-à-dire que selon un mode de réalisation préférentiel de l'invention, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.

Comme indiqué précédemment, ce composé peut comporter plus d'un motif à fonction amine. Il s'agit alors d'un polymère portant une répétition du motif à fonction amine.

Ce type de composés peut être représenté par l'une des formules suivantes :

HZ-(P)ₙ-ZH,

ou

HZ-(P')ₚ-ZH

dans lesquelles Z, P, P', n et p sont tels que définis plus haut.

A titre d'exemple de composé à fonction amine, on peut citer la N-méthyldiéthanolamine, la N-tert-butyl-diéthanolamine, la N-sulfoéthyldiéthanolamine.

Le deuxième composé entrant dans la préparation du polymère de formule I selon l'invention est un diisocyanate correspondant à la formule :

O=C=N-R₄-N=C=O

dans lequel R₄ est défini plus haut.

A titre d'exemple, on peut citer le méthylènediphényl-diisocyanate, le méthylènecyclohexanediisocyanate, l'isophorone-diisocyanate, le toluènediisocyanate, le naphtalènediisocyanate, le butanediisocyanate, l'hexanediisocyanate.

Un troisième composé entrant dans la préparation du polymère de formule I selon l'invention est un composé hydrophobe destiné à former les groupes hydrophobes terminaux du polymère de formule I.

Ce composé est constitué d'un groupe hydrophobe et d'une fonction à hydrogène labile, par exemple une fonction hydroxyle, amine primaire ou secondaire, ou thiol.

A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné α-hydroxyle.

Le groupe hydrophobe du composé de formule I selon l'invention peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.

Le polymère amphiphile associatif cationique de l'invention peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire, ou thiol. Ce composé peut être un polymère terminé aux extrémités des chaînes par l'une de ces fonctions à hydrogène labile.

A titre d'exemple, on peut citer, lorsqu'il ne s'agit pas de polymères, l'éthylèneglycol, le diéthylèneglycol et le propylèneglycol.

Lorsqu'il s'agit d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Le polymère préparé à partir des composés définis plus haut, est un polymère amphiphile associatif cationique de formule I selon la présente invention. Ce polymère est soluble ou dispersible dans l'eau et augmente de manière spectaculaire la viscosité de la solution aqueuse dans laquelle il est dissous ou dispersé.

Le groupe hydrophile noté Y dans la formule I est facultatif. En effet, les motifs à fonction amine quaternaire ou protonée peuvent suffire à apporter la solubilité ou l'hydrodispersibilité nécessaire pour ce type de polymère dans une solution aqueuse.

Bien que la présence d'un groupe Y hydrophile soit facultative, on préfère cependant des polyuréthannes amphiphiles associatifs cationiques comportant un tel groupe.

Etant donné ses bonnes propriétés épaississantes et son excellente affinité pour les matières kératiniques, ce type de polymère amphiphile associatif cationique selon l'invention est particulièrement adapté pour la préparation de compositions destinées à l'application topique à usage cosmétique.

En particulier, les polymères selon l'invention peuvent être utilisés dans des compositions capillaires, dans des compositions pour le soin de la peau, dans des compositions pour le soin des ongles, dans des compositions parfumantes, et dans des compositions de maquillage de la peau, des lèvres, des cils et des ongles.

A titre d'exemples, suivent ci-après des polymères amphiphiles associatifs cationiques de formule I, illustrant l'invention :

### Exemple 1 :

On synthétise le polymère suivant :
C₁₈H₃₇-O-CONHR₄NHCO-O-(CH₂)₂-N⁺(CH₃)(CH₃)-(CH₂)₂-O-CONHR₂NHCO-O(POE)O-CONHR₂NHCO-O-(CH₂)₂₋N⁺(CH₃)(CH₃)-(CH₂)₂-O-CONHR₄NHCO-OC₁₈H₃₇
   avec :
   contre ion : CH₃SO₄⁻
   R₄ = méthylènedicyclohexyle
   à partir des réactifs suivants :

| | |
|---|---|
| C₁₈H₃₇OH | 2 moles |
| Méthylènedicyclohexyldiisocyanate | 4 moles |
| Polyéthylèneglycol | 1 mole |
| N-méthyléthanolamine | 2 moles |
| Agent quaternisant (CH₃)₂SO₄ | 2 moles |

### Exemple 2 :

On synthétise le polymère suivant :
C₁₈H₃₇N⁺(CH₃)(CH₃)-(CH₂)₂-O-CONHR₄NHCO-O(POE)O-CONHR₄NHCO-O(CH₂)₂-N⁺(CH₃)(CH₃)C₁₈H₃₇ avec :
   R₄ = méthylènedicyclohexyle
   Contre ion : Cl⁻
   à partir des réactifs suivants :

| | |
|---|---|
| Méthylènedicyclohexyldiisocyanate | 2 moles |
| Polyéthylèneglycol | 1 mole |
| N,N-diméthyléthanolamine | 2 moles |
| Agent quaternisant C₁₈H₃₇OH | 2 moles |

### Exemple 3

### Réactifs:

| | |
|---|---|
| - polyoxyde d'éthylène (PEG) (Mₙ 10 000): | 0,010 mole |
| - méthylènedicyclohexyldiisocyanate : | 0,018 mole |
| - N,N-diméthyléthanolamine : | 0,020 mole |
| - bromure de stéaryle : | 0,024 mole |
| - octanoate d'étain (catalyseur) : | 0,2 % |

On solubilise 0,010 mole (100 g) de poly(oxyde d'éthylène) (PEG) ayant une masse moyenne en nombre de 10 000 dans 105 g de THF contenant 0,2 % d'octanoate d'étain (catalyseur), puis on ajoute goutte à goutte 0,018 mole (4,71 g) de méthylènedicyclohexyldiisocyanate. On chauffe le milieu réactionnel pendant 15 heures à reflux du THF en ajoutant après 6 heures 100 ml de THF. Pendant la réaction, on observe une disparition partielle de la bande NCO de l'isocyanate en FTIR et l'apparition des bandes CO et NH des liaisons amide formées. Le milieu est très visqueux et transparent.

On ajoute ensuite 0,020 moles (1,78 g) de N,N-diméthyléthanolamine et on laisse la réaction se poursuivre pendant 4 heures au reflux du THF jusqu'à disparition complète de la bande NCO et de la bande OH de l'alcool.

Pour la quaternisation, on ajoute au mélange réactionnel 0,024 mole (8 g) de bromure de stéaryle, c'est-à-dire un excès de 20 % en moles par rapport à la N,N-diméthyléthanolamine, puis 100 g de THF pour fluidifier le milieu réactionnel très visqueux. On poursuit le chauffage au reflux du THF pendant 36 heures supplémentaires.

On précipite le polymère obtenu dans de l'éther de pétrole, on filtre et on sèche sous vide à 40 °C pendant 24 heures. On obtient ainsi une poudre blanche friable.

On mesure par chromatographie par perméation de gel en milieu aqueux (étalonnage avec polystyrène) une masse moyenne en nombre de 70 000 et une masse moyenne en poids de 115 000, ce qui correspond à un indice de polydispersité de 1,65.

### Exemple 4

### Réactifs:

| | |
|---|---|
| - polyoxyde d'éthylène (PEG) (Mₙ 10 000) : | 0,010 mole |
| - méthylènedicyclohexyldiisocyanate : | 0,020 mole |
| - eau | 0,0334 mole |
| - bromure de stéaryle : | 0,022 mole |
| - dilaurate de dibutylétain (catalyseur) : | 0,5 % |

On sèche 100 g de polyéthylèneglycol pendant une nuit dans une étuve à une température de 100 °C en présence de P₂O₅. Le lendemain, on chauffe sous agitation mécanique le PEG à une température de 100 °C sous balayage d'argon, afin d'éliminer toute trace résiduelle d'eau. Cette opération dure 1 heure et 30 minutes. On ajoute ensuite goutte à goutte au PEG, 0,53 g de dilaurate de dibutylétain (catalyseur). Après 10 minutes, on ajoute 0,020 mole (5,24 g) de méthylènedicyclohexyldiisocyanate et on fait réagir pendant 1 heure et 30 minutes. On ajoute 105 g de toluène pour diminuer la forte viscosité du milieu. Après 22 heures de réaction, on ajoute 0,15 g de méthylènedicyclohexyldiisocyanate au mélange réactionnel et on poursuit la réaction pendant encore 2 heures.

On ajoute ensuite 0,0334 moles (6 g) d'eau et on poursuit la réaction pendant 8 heures en ajoutant 3 fois 105 g de toluène respectivement après 2 heures, 4 heures et 6 heures de réaction. A la fin de la réaction, on évapore le toluène.

Pour l'alkylation des fonctions amine primaire résultant de l'hydrolyse des groupes isocyanate aux extrémités du polymère, on dissout le polymère préparé ci-dessus dans 105 g de THF de manière à obtenir une solution à 50 % en poids, et on ajoute 0,022 moles (7,33 g) de bromure de stéaryle. On chauffe à reflux du THF pendant 20 heures. On isole le polymère par précipitation dans l'éther de pétrole, filtration et séchage sous vide à 40 °C pendant 24 heures. On obtient ainsi une poudre blanche friable.

On mesure par chromatographie par perméation de gel en milieu aqueux (étalonnage avec polystyrène) une masse moyenne en nombre de 52 000 et une masse moyenne en poids de 108 000, ce qui correspond à un indice de polydispersité de 2,07.

## Revendications

1. Polyuréthannes associatifs cationiques amphiphiles, hydrosolubles ou hydrodispersibles, de formule I :
R-X-(P)ₙ-[L-(Y)ₘ]ᵣ-L'-(P')ₚ-X'-R' (I)
dans laquelle :
R et R', identiques ou différents, représentent un groupement hydrophobe ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000 ;
la molécule contenant au moins une fonction amine protonée ou quaternisée et au moins deux groupements hydrophobes

2. Polyuréthannes selon la revendication 1, **caractérisés en ce que** les seuls groupements hydrophobes sont les groupes R et R' aux extrémités de chaîne.

3. Polyuréthannes selon l'une des revendications 1 ou 2, **caractérisés en ce que** R et R' représentent tous les deux indépendamment un groupement hydrophobe, X, X' représentent chacun un groupe L", n et p valent entre 1 et 1000 et L, L', L", P, P', Y et m ont la signification indiquée dans la revendication 1.

4. Polyuréthannes selon l'une des revendications 1 ou 2, **caractérisés en ce que** R et R' représentent tous les deux indépendamment un groupement hydrophobe, X, X' représentent chacun un groupe L", n et p valent 0 et L, L', L", Y et m ont la signification indiquée dans la revendication 1.

5. Polyuréthannes selon l'une des revendications 1 ou 2, **caractérisés en ce que** R et R' représentent tous les deux indépendamment un groupement hydrophobe, X et X' représentent tous les deux indépendamment un groupement comportant une amine quaternaire, n et p valent zéro, et L, L', Y et m ont la signification indiquée dans la revendication 1.

6. Polyuréthannes selon l'une des revendications précédentes, - **caractérisés en ce qu'**ils présentent une masse moléculaire moyenne en nombre comprise entre 400 et 500 000, de préférence entre 1000 et 400 000 et en particulier entre 1000 et 300 000.

7. Polyuréthannes selon l'une quelconque des revendications précédentes, **caractérisés en ce que** R et R' représentent un radical ou un polymère à chaîne hydrocarbonée, saturée ou non, linéaire ou ramifiée, dans laquelle un ou plusieurs des atomes de carbone peut être remplacé par un hétéroatome choisi parmi S, N, O et P, ou un radical à chaîne siliconée ou perfluorée.

8. Polyuréthannes selon l'une quelconque des revendications précédentes, **caractérisés en ce que** X et X' représentent l'une des formules : 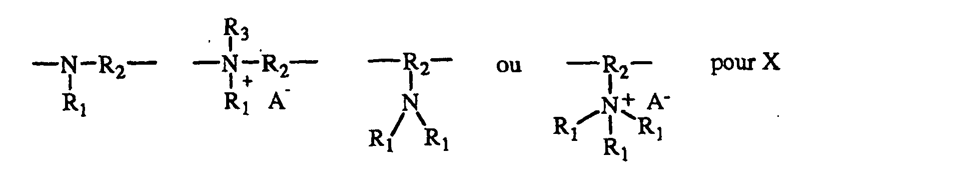 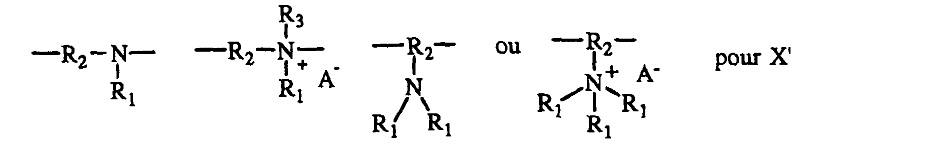 dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
A⁻ est un contre-ion physiologiquement acceptable.

9. Polyuréthannes selon l'une quelconque des revendications précédentes, **caractérisés en ce que** les groupements L, L' et L", identiques ou différents, représentent la formule : 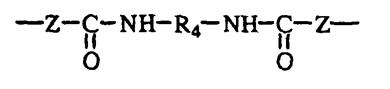 dans laquelle :
Z représente -O-, -S- ou -NH- ; et
R₄ représente un radical alkylène ayant de 1 à 20 atomes de carbone, linéaire ou ramifié, comportant ou non un cycle saturé ou non saturé, un radical arylène, un ou plusieurs des atomes de carbone pouvant être remplacé par un hétéroatome choisi parmi N, S, O et P.

10. Polyuréthannes selon l'une quelconque des revendications précédentes, **caractérisés en ce que** les groupements P et P', identiques ou différents, représentent au moins l'une des formules suivantes : 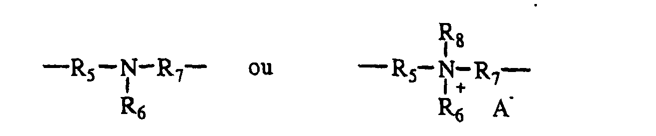 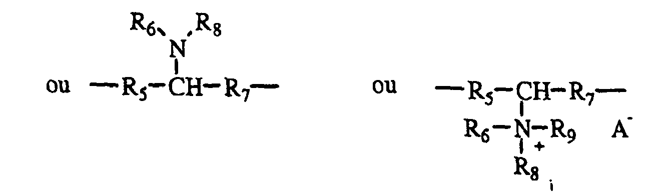 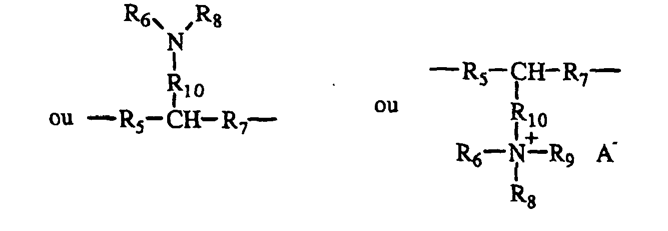 dans lesquelles :
R₅ et R₇ ont les mêmes significations que R₂ défini dans la revendication 7;
R₆, R₈ et R₉ ont les mêmes significations que R₁ et R₃ définis dans la revendication 7 ;
R₁₀ représente un groupe alkylène, linéaire ou ramifié, éventuellement insaturé et pouvant contenir un ou plusieurs hétéroatomes choisis parmi N, O, S et P, et
A- est un contre-ion physiologiquement acceptable.

11. Polyuréthannes selon l'une quelconque des revendications précédentes, **caractérisés par le fait que** Y représente un groupe dérivé d'éthylèneglycol, de diéthylèneglycol ou de propylèneglycol, ou un groupe dérivé d'un polymère choisi parmi les polyéthers, les polyesters sulfonés et les polyamides sulfonés.

12. Utilisation d'un polyuréthanne tel que défini dans l'une quelconque des revendications précédentes, à titre d'épaississant ou de gélifiant dans une composition pour application topique à usage cosmétique.

13. Composition cosmétique contenant, dans un milieu cosmétiquement acceptable, au moins un polyuréthanne tel que défini dans l'une quelconque des revendications 1 à 11.

## Claims

1. Water-soluble or water-dispersible amphiphilic cationic associative polyurethanes of formula (I):
R-X-(P)ₙ-[L-(Y)ₘ]ᵣ-L'-(P')ₚ-X'-R' (I)
in which:
R and R', which are identical or different, represent a hydrophobic group;
X and X', which are identical or different, represent a group comprising an amine functional group which may or may not carry a hydrophobic group or the L" group;
L, L' and L", which are identical or different, represent a group derived from a diisocyanate;
P and P', which are identical or different, represent a group comprising an amine functional group which may or may not carry a hydrophobic group;
Y represents a hydrophilic group;
r is an integer between 1 and 100, preferably between 1 and 50 and in particular between 1 and 25,
n, m and p have values, each independently of the others, between 0 and 1000;
the molecule comprising at least one protonated or quaternized amine functional group and at least two hydrophobic groups.

2. Polyurethanes according to Claim 1, **characterized in that** the only hydrophobic groups are the R and R' groups at the chain ends.

3. Polyurethanes according to either of Claims 1 and 2, **characterized in that** R and R' both independently represent a hydrophobic group, X and X' each represent an L" group, n and p have values between 1 and 1000 and L, L', L", P, P', Y and m have the meaning indicated in Claim 1.

4. Polyurethanes according to either of Claims 1 and 2, **characterized in that** R and R' both independently represent a hydrophobic group, X and X' each represent an L" group, n and p have the value 0 and L, L', L", Y and m have the meaning indicated in Claim 1.

5. Polyurethanes according to either of Claims 1 and 2, **characterized in that** R and R' both independently represent a hydrophobic group, X and X' both independently represent a group comprising a quaternary amine, n and p have the value 0 and L, L', Y and m have the meaning indicated in Claim 1.

6. Polyurethanes according to one of the preceding claims, **characterized in that** they exhibit a number-average molecular mass of between 400 and 500 000, preferably between 1 000 and 400 000 and in particular between 1 000 and 300 000.

7. Polyurethanes according to any one of the preceding claims, **characterized in that** R and R' represent a radical or a polymer with a saturated or unsaturated and linear or branched hydrocarbonaceous chain, in which chain one or more of the carbon atoms can be replaced by a heteroatom chosen from S, N, O and P, or a radical with a silicone or perfluorinated chain.

8. Polyurethanes according to any one of the preceding claims, **characterized in that** X and X' represent one of the formulae: 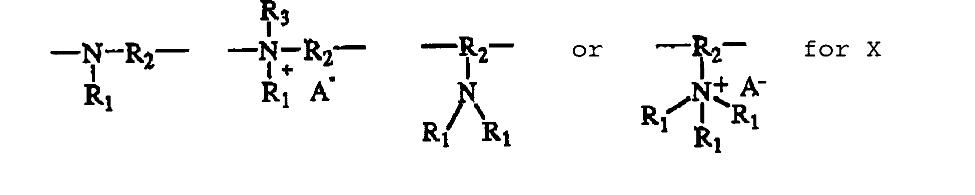 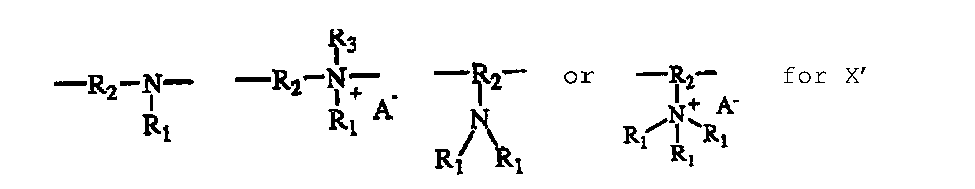 in which:
R₂ represents a linear or branched alkylene radical having from 1 to 20 carbon atoms, which may or may not comprise a saturated or unsaturated ring, or an arylene radical, it being possible for one or more carbon atoms to be replaced by a heteroatom chosen from N, S, O or P;
R₁ and R₃, which are identical or different, denote a linear or branched C₁-C₃₀ alkyl or alkenyl radical or an aryl radical, it being possible for at least one of the carbon atoms to be replaced by a heteroatom chosen from N, S, O or P;
A⁻ is a physiologically acceptable counterion.

9. Polyurethanes according to any one of the preceding claims, **characterized in that** the L, L' and L" groups, which are identical or different, represent the formula: 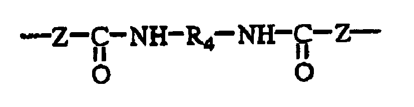 in which:
Z represents -O-, -S- or -NH-; and
R₄ represents a linear or branched alkylene radical having from 1 to 20 carbon atoms, which may or may not comprise a saturated or unsaturated ring, or an arylene radical, it being possible for one or more of the carbon atoms to be replaced by a heteroatom chosen from N, S, O and P.

10. Polyurethanes according to any one of the preceding claims, **characterized in that** the P and P' groups, which are identical or different, represent at least one of the following formulae: 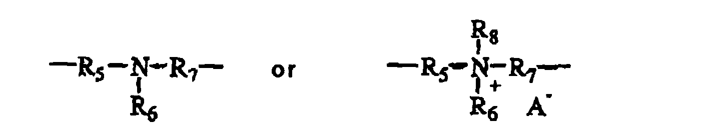 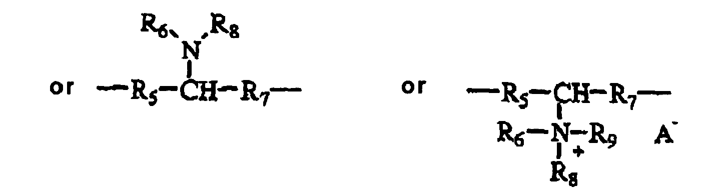 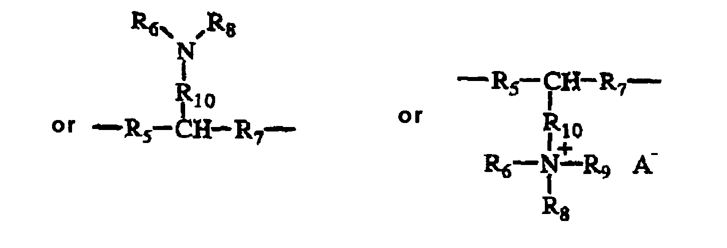 in which:
R₅ and R₇ have the same meanings as R₂ defined in Claim 7;
R₆, R₈ and R₉ have the same meanings as R₁ and R₃ defined in Claim 7;
R₁₀ represents a linear or branched alkylene group which is optionally unsaturated and which can comprise one or more heteroatoms chosen from N, O, S and P, and
A⁻ is a physiologically acceptable counterion.

11. Polyurethanes according to any one of the preceding claims, **characterized in that** Y represents a group derived from ethylene glycol, from diethylene glycol or from propylene glycol or a group derived from a polymer chosen from polyethers, sulphonated polyesters and sulphonated polyamides.

12. Use of a polyurethane as defined in any one of the preceding claims as thickener or gelling agent in a composition for topical application with a cosmetic use.

13. Cosmetic composition comprising, in a cosmetically acceptable medium, at least one polyurethane as defined in any one of Claims 1 to 11.

## Patentansprüche

1. Wasserlösliche oder wasserdispergierbare amphiphile kationische assoziative Polyurethane der Formel I:
R-X- (P)ₙ- [L-(Y)ₘ]ᵣ-L'-(P'ₚ-X'-R' (I)
worin:
R und R' gleich oder verschieden sind und für eine hydrophobe Gruppierung stehen;
X und X' gleich oder verschieden sind und für eine Gruppierung mit einer Aminfunktion, die gegebenenfalls eine hydrophobe Gruppierung trägt, oder auch die Gruppierung L" stehen;
L, L' und L" gleich oder verschieden sind und für eine Gruppierung, die sich von einem Diisocyanat ableitet, stehen;
P und P' gleich oder verschieden sind und für eine Gruppierung mit einer Aminfunktion, die gegebenenfalls eine hydrophobe Gruppierung trägt, stehen;
Y für eine hydrophile Gruppierung steht;
r für eine ganze Zahl zwischen 1 und 100, vorzugsweise zwischen 1 und 50 und insbesondere zwischen 1 und 25 steht;
n, m und p jeweils unabhängig voneinander Werte zwischen 0 und 1000 aufweisen;
wobei das Molekül mindestens eine protonierte oder quaternisierte Aminfunktion und mindestens zwei hydrophobe Gruppierungen enthält.

2. Polyurethane nach Anspruch 1, **dadurch gekennzeichnet, daß** die Gruppen R und R' an den Kettenenden die einzigen hydrophoben Grupppierungen sind.

3. Polyurethane nach Anspruch 1 oder 2, **dadurch gekennzeichnet, daß** R und R' beide unabhängig voneinander für eine hydrophobe Gruppierung stehen, X und X' jeweils für eine Gruppe L" stehen, n und p einen Wert zwischen 1 und 1000 aufweisen und L, L', L", P, P', Y und m die in Anspruch 1 angegebene Bedeutung besitzen.

4. Polyurethane nach Anspruch 1 oder 2, **dadurch gekennzeichnet, daß** R und R' beide unabhängig voneinander für eine hydrophobe Gruppierung stehen, X und X' jeweils für eine Gruppe L" stehen, n und p einen Wert von 0 aufweisen und L, L', L", Y und m die in Anspruch 1 angegebene Bedeutung besitzen.

5. Polyurethane nach Anspruch 1 oder 2, **dadurch gekennzeichnet, daß** R und R' beide unabhängig voneinander für eine hydrophobe Gruppierung stehen, X und X' beide unabhängig voneinander für eine Gruppierung, die ein quaternäres Amin umfaßt, stehen, n und p einen Wert von 0 aufweisen und L, L', Y und m die in Anspruch 1 angegebene Bedeutung besitzen.

6. Polyurethane nach einem der vorhergehenden Ansprüche, **dadurch gekennzeichnet, daß** sie ein zahlenmittleres Molekulargewicht zwischen 400 und 500.000, vorzugsweise zwischen 1000 und 400.000 und insbesondere zwischen 1000 und 300.000 aufweisen.

7. Polyurethane nach einem der vorhergehenden Ansprüche, **dadurch gekennzeichnet, daß** R und R' für einen Rest oder ein Polymer mit einer linearen oder verzweigten, gesättigten oder ungesättigten Kohlenwasserstoffkette, in der eines oder mehrere der Kohlenstoffatome durch ein unter S, N, O und P ausgewähltes Heteroatom oder einen Rest mit einer Silikonkette oder perfluorierten Kette ersetzt sein können, stehen.

8. Polyurethane nach einem der vorhergehenden Ansprüche, **dadurch gekennzeichnet, daß** X und X' für eine der Formeln: 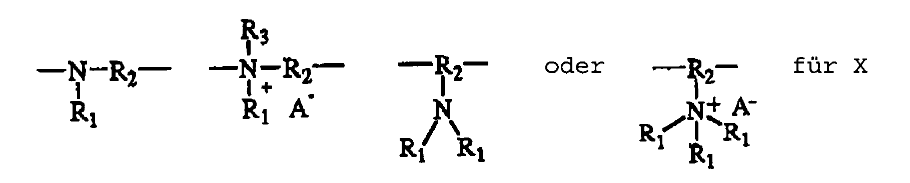 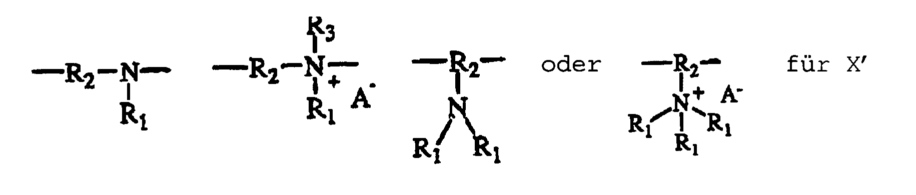 worin:
R₂ für einen linearen oder verzweigten Alkylenrest mit 1 bis 20 Kohlenstoffatomen, der gegebenenfalls einen gesättigten oder ungesättigten Ring umfaßt, oder einen Arylenrest steht, wobei ein oder mehrere Kohlenstoffatome durch ein unter N, S, O und P ausgewähltes Heteroatom ersetzt sein können;
R₁ und R₃ gleich oder verschieden sind und für einen linearen oder verzweigten C₁-C₃₀-Alkyl- oder -Alkenylrest oder einen Arylrest stehen, wobei mindestens eines der Kohlenstoffatome durch ein unter N, S, O und P ausgewähltes Heteroatom ersetzt sein kann;
A⁻ für ein physiologisch unbedenkliches Gegenion steht;
stehen.

9. Polyurethane nach einem der vorhergehenden Ansprüche, **dadurch gekennzeichnet, daß** die Gruppierungen L, L' und L" gleich oder verschieden sind und für die Formel: 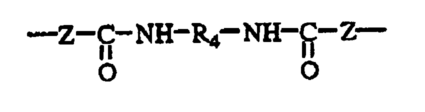 worin:
Z für -O-, -S- oder -NH- steht und
R₄ für einen linearen oder verzweigten Alkylenrest mit 1 bis 20 Kohlenstoffatomen, der gegebenenfalls einen gesättigten oder ungesättigten Ring umfaßt, oder einen Arylenrest steht, wobei ein oder mehrere Kohlenstoffatome durch ein unter N, S, O und P ausgewähltes Heteroatom ersetzt sein können; stehen.

10. Polyurethane nach einem der vorhergehenden Ansprüche, **dadurch gekennzeichnet, daß** die Gruppierungen P und P' gleich oder verschieden sind und für mindestens eine der folgenden Formeln stehen: 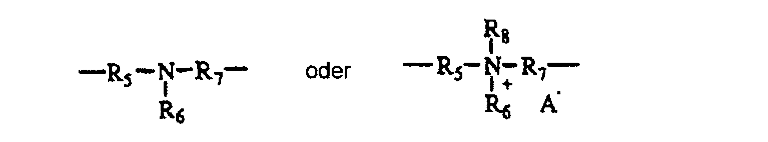 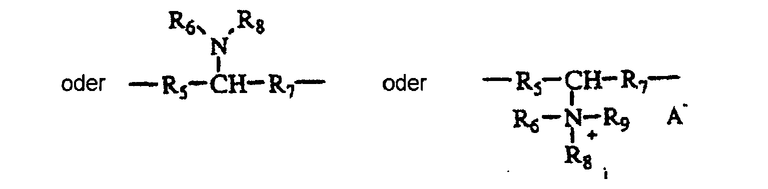 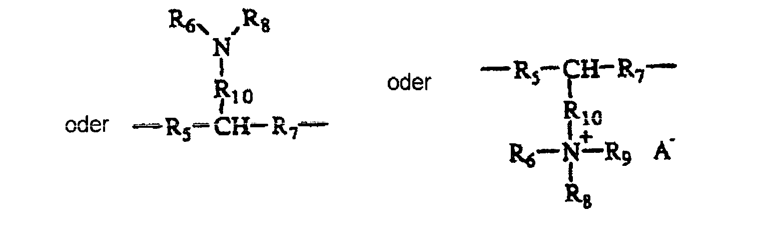 worin:
R₅ und R₇ die gleichen Bedeutungen wie R₂ gemäß Anspruch 7 besitzen;
R₆, R₈ und R₉ die gleichen Bedeutungen wie R₁ und R₃ gemäß Anspruch 7 besitzen;
R₁₀ für eine gegebenenfalls ungesättigte lineare oder verzweigte Alkylengruppe, die ein oder
mehrere unter N, O, S und P ausgewählte Heteroatome enthalten kann, steht und
A⁻ für ein physiologisch unbedenkliches Gegenion steht.

11. Polyurethane nach einem der vorhergehenden Ansprüche, **dadurch gekennzeichnet, daß** Y für eine Gruppe, die sich von Ethylenglykol, Diethylenglykol oder Propylenglykol ableitet, oder eine Gruppe, die sich von einem unter Polyethern, sulfonierten Polyestern und sulfonierten Polyamiden ausgewählten Polymer ableitet, steht.

12. Verwendung eines Polyurethans gemäß einem der vorhergehenden Ansprüche als Verdickungs- oder Gelierungsmittel in einer Zusammensetzung zur topischen Anwendung bei einer kosmetischen Verwendung.

13. Kosmetische Zusammensetzung, die mindestens ein Polyurethan gemäß einem der Ansprüche 1 bis 11 in einem kosmetisch unbedenklichen Medium enthält.
